# EUROPÄISCHE PATENTSCHRIFT

(11) **EP 2 634 115 B1**
(45) Veröffentlichungstag und Bekanntmachung des Hinweises auf die Patenterteilung: **03.09.2014**
(21) Anmeldenummer: 12157483.4
(22) Anmeldetag: 29.02.2012
(51) Int. Cl.: B65G 1/04

(54) **Verfahren zum Betreiben einer Apothekenkommissioniervorrichtung**
Method for operating a pharmacy picking device
Procédé de fonctionnement d'un dispositif de commissionnement de pharmacie

(43) Veröffentlichungstag der Anmeldung: 04.09.2013
(73) Patentinhaber: CareFusion Germany 326 GmbH, 53539 Kelberg (DE)
(72) Erfinder: Hellenbrand, Christoph, 56761 Kaifenheim (DE); klapperich, Andreas, 59745 Rieden (DE); Bause, Andreas, 56642 Kruft (DE); Reif, Dennis, 56759 Kaisersesch (DE)
(74) Vertreter: Zenz

(56) Entgegenhaltungen:
- EP-A2- 1 035 044
- CH-A- 520 612
- FR-A1- 2 608 567
- JP-A- 60 183 405
- JP-A- 61 060 504
- US-A- 3 504 245
- US-A- 4 415 975
- US-A1- 2008 044 262

## Beschreibung

Die vorliegende Erfindung betrifft ein Verfahren zum Betreiben einer Apothekenkommissioniervorrichtung mit zumindest einer Regalreihe mit jeweils einer Mehrzahl von sich in einer horizontalen Richtung (X-Achse) erstreckenden Regalböden und einer Mehrzahl von sich in einer vertikalen Richtung (Z-Achse) erstreckenden Regalwänden, zumindest einem vor der Regelreihe horizontal und vertikal verfahrbaren Bediengerät mit einer Greifvorrichtung zum Ein- und/oder Auslagern von Arzneimittelpackungen auf die bzw. von den Regalböden, wobei das Bediengerät zumindest einen Sensor aufweist, und mit einer mit dem Bediengerät gekoppelten Steuereinrichtung.

In modernen Apothekenkommissioniervorrichtungen wird eine große Anzahl verschiedener und unterschiedlich dimensionierter Arzneimittelpackungen (Stückgüter) chaotisch und platzoptimiert auf langgestreckten Regalböden gelagert. Die gelagerten Arzneimittelpackungen werden mit Hilfe eines Bediengerätes ein- bzw. ausgelagert, wobei das Bediengerät eine Greifvorrichtung zum Ergreifen von Arzneimittelpackungen aufweist. Zur Einlagerung der Arzneimittelpackungen werden die Arzneimittelpackungen identifiziert, vermessen (d. h. die Abmessungen in den drei Dimensionen bestimmt) und in einen Greifbereich der Greifvorrichtung eines Regalbediengeräts transportiert (vgl. z.B. DE 195 09 951 C2).

Anhand der Abmessungen der einzulagernden Arzneimittelpackungen sowie der Belegung der Apothekenkommissioniervorrichtung werden optimale Ablageorte für die Arzneimittelpackungen berechnet und in der Steuereinrichtung abgespeichert. Zur Berechnung eines optimalen Ablageorts ist es ferner erforderlich, dass die Steuereinrichtung den genauen geometrischen Aufbau der Apothekenkommissioniervorrichtungen und insbesondere die Lage bzw. Anordnung und Dimensionierung der Regalböden und Regalwände kennt; nur so kann beispielsweise verhindert werden, dass als Ablageort eine Position innerhalb der Apothekenkommissioniervorrichtung gewählt wird, die beispielsweise von einer Regalwand "belegt" ist. Der Ablageort für eine Arzneimittelpackung wird als Raumkoordinaten (mit einem X-, Y-, Z-Achsenanteil) innerhalb der Apothekenkommissioniervorrichtung berechnet. Als Bezugspunkt (Ursprung) für diese Raumkoordinaten kann beispielsweise ein bestimmter Referenzpunkt innerhalb der Vorrichtung dienen (beispielsweise bestimmte Position des Bediengerätes / der Greifvorrichtung); es ist jedoch auch denkbar, dass beispielsweise jedes aus Regalböden und Regalfächern gebildete Regalfach einen eigenen Bezugspunkt aufweist. Die Lage (bzw. die Raumkoordinaten) einer Arzneimittelpackung ist dann genauso eindeutig über die Raumkoordinaten des Bezugspunktes des Faches und die Raumkoordinate der Arzneimittelpackung innerhalb des Regalfaches bestimmt.

Zur weiteren Einlagerung ergreift die Greifvorrichtung die Arzneimittelpackungen in dem Greifbereich und transportiert diese zu dem vorbestimmten Ablageort. Zur optimalen Raumausnutzung werden die Regalböden dicht belegt, wobei nebeneinander Arzneimittelpackungen unterschiedlicher Abmessungen und unterschiedlicher Art gelagert werden können.

Wenn - beispielsweise aufgrund eines Wunsches eines Kunden der Apotheke - eine bestimmte Arzneimittelpackung ausgelagert werden soll, so steuert das Bediengerät den Ablageort der gewünschten Arzneimittelpackung an, ergreift mit der Greifvorrichtung die Arzneimittelpackung und gibt diese an eine Ausgabestelle bzw. an eine Transporteinrichtung, die die Arzneimittelpackung zu einer Ausgabestelle (beispielsweise im Verkaufsraum einer Apotheke) transportiert, ab. Je nach baulichen Gegebenheiten muss kein Transport erfolgen.

Die Genauigkeit der Positionierung der Bediengeräts und/oder der Greifvorrichtung bei der Einlagerung/Auslagerung ist für einen reibungslosen Betrieb der Apothekenkommissioniervorrichtung wesentlich. Bei der Einlagerung beispielsweise wird von der Steuereinrichtung anhand der Geometrie der Apothekenkommissioniervorrichtung, der Abmessungen der einzulagernden Arzneimittelpackung sowie der Belegung der Vorrichtung ein Ablageort mit bestimmten Raumkoordinaten errechnet. Dieser ist von dem Bediengerät anzufahren, und die Arzneimittelpackung ist bei dem berechneten Ablageort einzulagern. Eine nicht präzise Positionierung des Bediengerätes kann eine fehlerfreie Einlagerung verhindern (wenn beispielsweise eine Position mit einer Regalwand oder eine bereits belegte Regalposition angefahren wird). Bezogen auf das Auslagern bedeutet eine unpräzise Positionierung des Bediengerätes und der Greifvorrichtung, dass unter Umständen eine Auslagerung einer bestimmten Arzneimittelpackung mit dem Bediengerät nicht möglich ist und Arzneimittelpackungen ggf. händisch entnommen werden müssen, was zu ungewünschten Verzögerungen und einer Störung des automatischen Betriebs führt.

Das Bediengerät selber ist eine komplexe mechanische Vorrichtung mit einer Mehrzahl von Antriebseinrichtungen, die ein Verfahren des Bediengerätes und der Greifvorrichtung in horizontaler und vertikaler Richtung (X- bzw. Z-Achse) ermöglichen. Im Betrieb der Apothekenkommissioniervorrichtung ist das Bediengerät und die Greifvorrichtung hohen Belastungen ausgesetzt und dies kann dazu führen, dass die Genauigkeit, mit welcher das Bediengerät in Bezug auf einen gegebenen Ort innerhalb der Vorrichtung positioniert werden kann, schleichend nachlässt.

Wenn die Antriebseinrichtung für die Positionierung in X-Richtung beispielsweise einen oder mehrere Zahnriemen umfasst, können sich diese mit der Zeit dehnen, d.h. es tritt eine Längenänderung des Zahnriemens auf. Wenn der gedehnte Zahnriemen mit einem entsprechenden Motor (beispielsweise einem Schrittmotor) nun um die gleiche Anzahl von Schritten zum Ablageort verfahren wird, die zur Positionierung bei nichtgedehntem Zahnriemen notwendig sind, hat dies zur Folge, dass die Positionierung in X-Richtung fehlerhaft ist (die eigentliche Position ist aufgrund der Längendehnung des Zahnriemens noch gar nicht erreicht). In Abhängigkeit von der genauen Konstruktion der Antriebseinrichtungen können noch andere/weitere Probleme eine präzise Positionierung verhindern. Eine unpräzise Positionierung des Bediengerätes bedingt demnach eine Störung automatischen (Dauer)Betrieb einer Apothekenkommissioniervorrichtung, da die Gefahr besteht, dass Arzneimittelpackungen mit dem Bediengerät nicht ein- ausgelagert werden können.

Aus dem Stand der Technik sind bereits Verfahren zum Positionieren Bediengeräten bekannt. So beschreibt beispielsweise die FR 2 608 567 A1 ein Verfahren und eine Vorrichtung zum Steuern eines Hubwagens, der dazu dient, Waren in einem Hochregallager zu transportieren und zu verstauen. Das Lager wird vorzugsweise automatisch bedient und ist aus Fächern, Regalen oder Palettenträgern aufgebaut, die von vertikalen Säulen oder Ständern abgestützt werden. Erfindungsgemäß werden die Vorderflächen der Fächer mit Messpunkten versehen, die zum Erlernen der Positionen der einzelnen Fächer dienen.

US 3 504 245 beschreibt ein Verfahren zur automatischen Positionierung eines Bediengeräts, wobei die Positionierung durch Abtasten von Markierungen realisiert wird, die an Führungen für das Bediengerät angebracht sind.

EP 1 035 044 A2 offenbart eine Vorrichtung zur Positionierung eines Bediengeräts vor einem Regalfachsystem. An dem Regalfachsystem sind Kodierungen aufweisende Marker angebracht, die während einer Referenzfahrt von einem Sensor an dem Bediengerät erfasst und als Referenzwerte gespeichert werden Zur Positionierung des Bediengeräts wird dieses an dem Regalfachsystem vorbeigeführt, bis mit dem Sensor der "richtige" Marker ermittelt ist. Dann beginnt die Feinpositionierung, welche mittels einer Zielmarke durchgeführt wird, die mittig an einem Regalboden angeordnet ist.

Aufgabe der Erfindung ist es, ein Verfahren zum Betreiben einer Apothekenkommissioniervorrichtung bereitzustellen, welches die Störanfälligkeit des automatischen Betriebes der Apothekenkommissioniervorrichtung vermindert.

Diese Aufgabe wird erfindungsgemäß durch ein Verfahren mit den Merkmalen des Patentanspruchs 1 gelöst.

Die Apothekenkommissioniervorrichtung, welche mit dem erfindungsgemäßen Verfahren betrieben wird, umfasst zumindest eine Regalreihe mit jeweils einer Mehrzahl von sich in einer horizontalen Richtung erstreckenden Regalböden und einer Mehrzahl von sich in einer vertikalen Richtung erstreckenden Regalwänden, zumindest ein vor der Regalreihe horizontal und vertikal verfahrbaren Bediengerät mit einer Greifvorrichtung zum Ein- und/oder Auslagern von Arzneimittelpackungen auf die bzw. von den Regalböden, wobei das Bediengerät zumindest einen Sensor aufweist, und eine mit dem Bediengerät gekoppelte Steuereinheit, die sämtliche Arbeitsabläufe innerhalb der Apothekenkommissioniervorrichtung steuert.

Bei dem erfindungsgemäßen Verfahren zum Betreiben einer Apothekenkommissioniervorrichtung wird zum Erkennen einer Positionierungsabweichung des Bediengerätes in horizontaler Richtung (X-Achse) zunächst zumindest ein Sollwert zumindest einer Referenzposition bereitgestellt.

Eine Referenzposition hinsichtlich der X-Achse kann beispielsweise die Lage einer bestimmten Regalwand innerhalb der Apothekenkommissioniervorrichtung sein. Der Sollwert beschreit dann den Abstand der Referenzposition zu einem Bezugspunkt innerhalb der Apothekenkommissioniervorrichtung. Werden zwei Sollwerte von zwei Referenzpositionen bereitgestellt, können die Referenzpositionen den Anordnungen von zwei Regalwänden innerhalb der Apothekenkommissioniervorrichtung entsprechen. Als Referenzpositionen können auch andere Punkte gewählt werden; wichtig ist lediglich, dass diese im Laufe des Betriebes keine räumliche Veränderung erfahren.

Bedingt durch die Art der verbauten Antriebseinrichtung für die Bewegung des Bediengeräts in X-Richtung sowie deren Charakteristika ist es der Steuereinrichtung bekannt, wie bei einer "fehlerfreien" (d.h. mit "Ursprungscharakteristika") Antriebseinrichtung eine Referenzposition mit vorgegebener Präzision anzufahren ist (mit beispielsweise X Motorschritten bei Verwendung eines Schrittmotors).

Bei dem erfindungsgemäßen Verfahren wird dann mit dem Bediengerät eine einem Sollwert entsprechende Position in horizontaler Richtung angefahren, wobei die vorgenannte Position von dem Sollwert der Referenzposition abweicht, d.h. es wird eine Position hinter oder vor der eigentlichen Referenzposition angefahren.

Das Bediengerät wird mit eingeschaltetem Sensor (der Sensor kann erst bei der dem Sollwert entsprechenden Position eingeschaltet werden oder befindet sich im Dauerbetriebszustand) weiter in horizontaler Richtung auf den eigentlichen Sollwert hin verfahren und bei Erfassen eines für die Referenzposition charakteristischen Signals durch den Sensor wird ein Istwert dieser Referenzposition ermittelt. Bei dem Sensor kann es sich beispielsweise um einen optischen Sensor Optischen Sensor nach dem Triangulationsprinzip) handeln, der einen Abstand des Sensors zu einem Gegenstand ermitteln kann, an welchem der Sensor vorbeigeführt wird. Der ermittelte Istwert gibt die ermittelten Raumkoordinaten (oder zumindest den X-Anteil davon) der Referenzposition zum Zeitpunkt der Ermittlung des Istwertes wieder.

Anschließend wird ein Sollwert mit einem entsprechenden Istwert verglichen, oder es werden zwei Istwerte miteinander verglichen, und eine Abweichung bestimmt. Abgesehen von zu vernachlässigender Wärmedehnung ändert sich die absolute Lage der Referenzposition(en) innerhalb der Apothekenkommissioniervorrichtung nicht. Im Idealfall (der beispielsweise dem Urzustand entspricht, bei welchem die Sollwerte ermittelt wurden) ist der Istwert mit dem Sollwert identisch. Wird aber eine Abweichung ermittelt, bedeutet dies, dass zum Erreichen/Anfahren der Referenzposition(en) in X-Richtung (bei Verwendung eines Schrittmotors beispielsweise) eine andere Anzahl von Motorschritten benötigt wurde. Die Positionierung ist also dann, wenn die Steuereinheit ein Steuerprogramm fährt, welches eine fehlerfreie bzw. ungestörte Antriebseinrichtung(en) voraussetzt (X Motorschritte zum Erreichen einer bestimmten Referenzposition), nicht mehr präzise (bei ungestörter Antriebeinrichtung werden X Motorschritte zum Erreichen der Referenzposition benötigt, mit gestörter X+ΔX oder X-ΔX Motorschritte; ohne Kenntnis der Störung wird also eine um ΔX Schritte falsche Position angefahren).

Werden zwei Istwerte miteinander verglichen so besagt eine Abweichung, dass für das Zurücklegen der Entfernung zwischen den beiden Referenzpunkten in X-Richtung (bei Verwendung eines Schrittmotors beispielsweise) eine andere als die erwartete Anzahl von Motorschritten benötigt wurde. Auch aus dieser Abweichung ergibt sich, dass die Positionierung auf der Basis des in der Steuereinheit vorhandenen Steuerprogramms nicht mehr präzise ist.

Sofern eine einen Grenzwert überschreitende Abweichung ermittelt wird, wird dann ein auf die Notwendigkeit einer Korrektur hinweisendes Signal ausgegeben.

Das erfindungsgemäße Verfahren vermindert die Notwendigkeit einer händischen Entnahme einer Arzneimittelpackung und vermindert die Störanfälligkeit, indem in vorgegebenen Abständen geprüft wird, ob die Positionierung in X-Richtung noch ausreichend präzise ist. Ist dies nicht der Fall, wird ein entsprechendes Signal ausgeben, wobei das Signal in Abhängigkeit von beispielsweise der Art und der Größe der Abweichung variieren kann. Bei lediglich geringen Abweichungen kann eine interne Anpassung vorgenommen werden, die eine Fortführung des automatischen Betriebes gewährleistet.

Mit der X-Richtung/X-Achse wird bei einer Apothekenkommissioniervorrichtung üblicherweise die Richtung bezeichnet, in der das Bediengerät horizontal verfährt. Aufgrund der Art der Einlagerung der Arzneimittelpackungen ist die X-Achse regelmäßig auch die längste Achse, was zur Folge hat, dass das Bediengerät entlang dieser Achse besonders häufig verfahren wird und sich Abweichungen in der Positionierungsgenauigkeit besonders stark auswirken.

Bei einer bevorzugten Ausführungsform des erfindungsgemäßen Verfahrens wird zusätzlich zum Erkennen einer Positionierungsabweichung in horizontaler Richtung (X-Achse) die Z-Achse (vertikale Achse) hinsichtlich einer Positionierungsabweichung untersucht.

Dazu wird zumindest ein Sollwert zumindest einer Referenzposition bereitgestellt. Dabei kann es sich beispielsweise um die Lage eines oder mehrerer Regalböden handeln. Dann wird mit dem Bediengerät eine einem Sollwert entsprechende Position angefahren und bei eingeschaltetem Sensor die Greifvorrichtung weiter auf den Sollwert bewegt und bei Erfassen eines für eine Referenzposition charakteristischen Signals ein Istwert dieser Referenzposition ermittelt. Ein Sollwert wird mit einem entsprechenden Istwert, oder zwei Istwerte miteinander, verglichen und eine Abweichung wird bestimmt. Sofern eine einen Grenzwert überschreitende Abweichung ermittelt wird, wird ein auf die Notwendigkeit einer Korrektur hinweisendes Signal ausgegeben, wobei dieses Signal mit der Art und der Größe der Abweichung variieren kann.

Aufgrund der enormen Vielfalt an Arzneimitteln ist es notwendig, stets eine Vielzahl verschiedener Arzneimittelpackungen vorrätig zu haben; dazu ist ein entsprechend großer Lagerplatz innerhalb der Apothekenkommissioniervorrichtung erforderlich. Dies kann man beispielsweise erreichen, indem man die Vorrichtung in X- und/oder Z-Richtung erweitert. Eine andere Möglichkeit ist, zwei meist parallele Regalreichen zu verbauen. Jeder Regalreihe kann ein Bediengerät zugeordnet sein, jedoch ist es aufgrund der kostenintensiven Komponenten eines Bediengerätes bevorzugt, lediglich ein Bediengerät zu verbauen. Um beide Regalreihen bedienen zu können, ist es erforderlich, dass die Greifvorrichtung des Bediengerätes zumindest um 180° drehbar ist (C-Achse), so dass beide Regalreihen von vorne zum Ein- und Auslagern bedient werden können. Auch bei der Drehung um die C-Achse kann es zu Positionierungsabweichungen kommen, beispielsweise derart, dass nicht um 180°C, sondern um 180°+X° oder 180°-X° gedreht wird (beispielsweise aufgrund eines Schlupfes des Drehmotors bei zu geringer Drehung).

Bei einer bevorzugten Ausführungsform des erfindungsgemäßen Verfahrens wird daher zusätzlich die Drehung um die C-Achse überprüft. Die Apothekenkommissioniervorrichtung, welche mit der bevorzugten Ausführungsform des erfindungsgemäßen Verfahrens betrieben wird, umfasst zumindest zwei parallele Regalreihen, zwischen denen das Bediengerät horizontal und vertikal verfahrbar ist, wobei die Greifvorrichtung des Bediengerätes um die Vertikalachse drehbar bzw. schwenkbar ist.

Zum Erkennen einer Positionierungsabweichung der Greifvorrichtung bezüglich der C-Drehachse wird zunächst, wie dies bereits oben beschrieben wurde, eine Überprüfung hinsichtlich der Positionierungsabweichung in X-Richtung bezüglich einer der Regalreihen durchgeführt. Die Greifvorrichtung wird dann um einen vorgegebenen Wert C gedreht, wobei dieser Wert im Idealfall (ohne Störung der Drehung um die C-Drehachse) eine Drehung um 180° bewirkt.

Anschließend wird überprüft, ob bei der zweiten Regalreihe eine Positionierungsabweichung in X-Richtung vorliegt. Dazu wird bei der zweite Regalreihe mit dem Bediengerät eine zweite, einem zweiten Sollwert entsprechende Position angefahren (es wird also pro Regalreihe zumindest eine Referenzposition in X-Richtung bereitgestellt), bei eingeschaltetem Sensor die Greifvorrichtung weiter auf den Sollwert verfahren und bei Erfassen eines für die zweite Referenzposition charakteristischen Signals ein Istwert der Referenzposition ermittelt. Der Sollwert der zweiten Referenzposition wird mit dem Istwert verglichen und eine Abweichung wird bestimmt. Schließlich wird, sofern die ermittelten Abweichungen hinsichtlich der Positionierung in X-Richtung in Bezug auf die erste und die zweite Regalreihe Grenzwerte überschreiten oder sich um einen vorgegebenen Wert unterscheiden, ein die Notwendigkeit einer Korrektur anzeigendes Signal ausgegeben, wobei das Signal in Abhängigkeit von den ermittelten Abweichungen variiert.

Abweichungen bei der Positionierungsgenauigkeit hinsichtlich der C-Achse werden also nicht direkt an dieser ermittelt, sondern indirekt über Positionierungsabweichungen in Bezug auf die X-Achse der Regalreihen.

Das Bediengerät ist üblicherweise mit Hilfe von Führungsschienen in horizontaler und vertikaler Richtung verfahrbar. In Abhängigkeit von der Gestaltung der Apothekenkommissioniervorrichtung werden zwei horizontale Führungsschienen verwendet, an welchen die vertikale Führungsschien mit der Greifvorrichtung horizontal verfahren wird. Jeder Führungsschiene kann eine eigenständige Antriebseinrichtung zugeordnet sein (oder ein Abschnitt einer zentralen Antriebseinrichtung), und die Antriebseinrichtungen sorgen für eine (möglichst synchrone) Bewegung entlang der horizontalen Führungsschienen.

Wenn die Antriebseinrichtungen die vertikale Führungsschiene nicht synchron bewegen, kann dies zu einer Schrägstellung der vertikalen Führungsschiene führen und eine solche Schrägstellung beeinflusst wiederrum die Positionierungsgenauigkeit des Bediengerätes und der Greifvorrichtung.

Bei einer bevorzugten Ausführungsform des erfindungsgemäßen Verfahrens wird zum Erkennen einer Schrägstellung des Bediengerätes für die untere und die obere Führungsschiene zumindest ein Sollwert zumindest einer Referenzposition bereitgestellt, mit dem Bediengerät eine den Sollwerten entsprechende Position angefahren, bei Erfassen eines für eine Referenzposition charakteristischen Signals ein Istwert pro Referenzpositionen ermittelt und die Sollwerte mit den Istwerten verglichen und eine Abweichungen pro Referenzposition bestimmt. Schließlich wird, sofern sich die ermittelten Abweichungen um mehr als einen vorgegebenen Differenzwert unterscheiden, ein die Notwendigkeit einer Korrektur anzeigendes Signal ausgegeben, wobei dieses Signal von der Größe des Differenzwertes abhängig ist.

Bei einer bevorzugten Ausführungsform des erfindungsgemäßen Verfahrens wird in Abhängigkeit von dem die Notwendigkeit einer Korrektur anzeigenden Signals eine Serviceanfrage elektronisch an einen Servicemitarbeiter übermittelt und/oder die Apothekenkommissioniervorrichtung abgeschaltet. Eine Serviceanfrage wird immer dann übermittelt, wenn die festgestellten Abweichungen/Differenzen zwar vorgegebene Grenzwerte überschreiten, eine Fehlfunktion der Anlage aber noch weitgehend ausgeschlossen werden kann. Auf diese Weise kann bereits eine Wartung der Anlage in die Wege geleitet werden, wenn das Abschalten der Anlage noch nicht erforderlich ist. Ein Tätigwerden des Benutzers ist dann noch nicht erforderlich.

Der Sollwert bzw. die Sollwerte können bereitgestellt werden, indem der Sollwert bzw. die Sollwerte in der Steuereinrichtung als vorgegebene Werte hinterlegt werden. Diese Vorgehensweise ist ausgesprochen einfach und rasch durchführbar, erfordert jedoch ein sehr hohes Maß an Fertigungsgenauigkeit. Es ist bevorzugt, dass der Sollwert bzw. die Sollwerte zumindest einer Referenzposition bereitgestellt wird bzw. werden, indem der Sollwert bzw. die Sollwerte nach beispielsweise der ersten Inbetriebnahme der Apothekenkommissioniervorrichtung erlernt werden, indem vorgegebene Referenzpositionen angefahren werden und bei Erfassen eines für eine bestimmte Referenzposition charakteristischen Signals ein Wert ermittelt und dieser Wert als Sollwert und in der Steuereinrichtung gespeichert wird.

Insbesondere bei Großapotheken ist die Apothekenkommissio-niervorrichtung quasi im Dauerbetrieb, so dass es wünschenswert ist, dass das erfindungsgemäße Verfahren möglichst rasch durchführbar ist. Bei einer bevorzugten Ausführungsform des erfindungsgemäßen Verfahrens wird daher eine einem Sollwert entsprechende Position mit einer ersten Geschwindigkeit v1 angefahren und das Bediengerät mit einer zweiten Geschwindigkeit v2 weiter in Richtung auf die Referenzposition verfahren, bis ein für die Referenzposition charakteristischen Signal erfasst wird, wobei die Geschwindigkeit v2 kleiner als die Geschwindigkeit v1 ist.

Das Bediengerät kann beispielsweise mit voller Geschwindigkeit zu der einem Sollwert entsprechenden Position verfahren werden; dort kann dann, sofern der Sensor nicht im Dauerbetrieb arbeitet, der Sensor angeschaltet werden. Dann wird das Bediengerät mit verminderter Geschwindigkeit weiterbewegt, bis ein für die Referenzposition charakteristischen Signal erfasst wird. Die verminderte Geschwindigkeit trägt zur Erhöhung der Messgenauigkeit bei und die Kombination mit einer Wegstrecke mit voller Geschwindigkeit ermöglicht ein rasches und zugleich zuverlässiges Ausführen des Verfahrens.

Die Positionierungsabweichungen können vielfältige Gründe haben (Details folgen in der Figurenbeschreibung), so dass aus ermittelten Abweichungen nicht stets zwangsläufig auf die Ursache geschlossen werden kann. Sofern jedoch aufgrund der ermittelten Abweichungen eindeutig ist, wodurch die Positionierungsabweichung bedingt ist, werden bei einer bevorzugten Ausführungsform des erfindungsgemäßen Verfahrens auf der Basis des oder der ermittelten Abweichung(en) ein oder mehrere entsprechende Korrekturfaktoren ermittelt, diese in der Steuereinrichtung gespeichert und nachfolgend ausgeführte Steuerprogramme berücksichtigen bei dem Ansteuern des Bediengerätes den bzw. die Korrekturfaktoren.

Im Nachfolgenden wir das erfindungsgemäße Verfahren anhand von in der Zeichnung zum Teil lediglich schematisch dargestellten bevorzugten Ausführungsformen näher beschrieben. In der Zeichnung zeigen:
Figur 1 eine seitliche Schnittansicht einer Regalreihe einer Apothekenkommissioniervorrichtung,
Figur 2 eine Draufsicht auf eine Apothekenkommissioniervorrichtung,
Figur 3 eine Schnittansicht einer Apothekenkommissioniervorrichtung,
Figuren 4a-4c Schnittansichten eines Abschnitts einer Apothekenkommissioniervorrichtung, wobei die Figuren zur Veranschaulichung des erfindungsgemäßen Verfahrens dienen sollen,
Figuren 5a-5b Schnittansichten eines Abschnitts einer Apothekenkommissioniervorrichtung, wobei die Figuren zur Veranschaulichung des erfindungsgemäßen Verfahrens dienen sollen, und
Figur 6 eine schematische Ansicht eines Abschnittes einer Apothekenkommissioniervorrichtung, wobei die Figur zur Veranschaulichung des erfindungsgemäßen Verfahrens dienen soll.

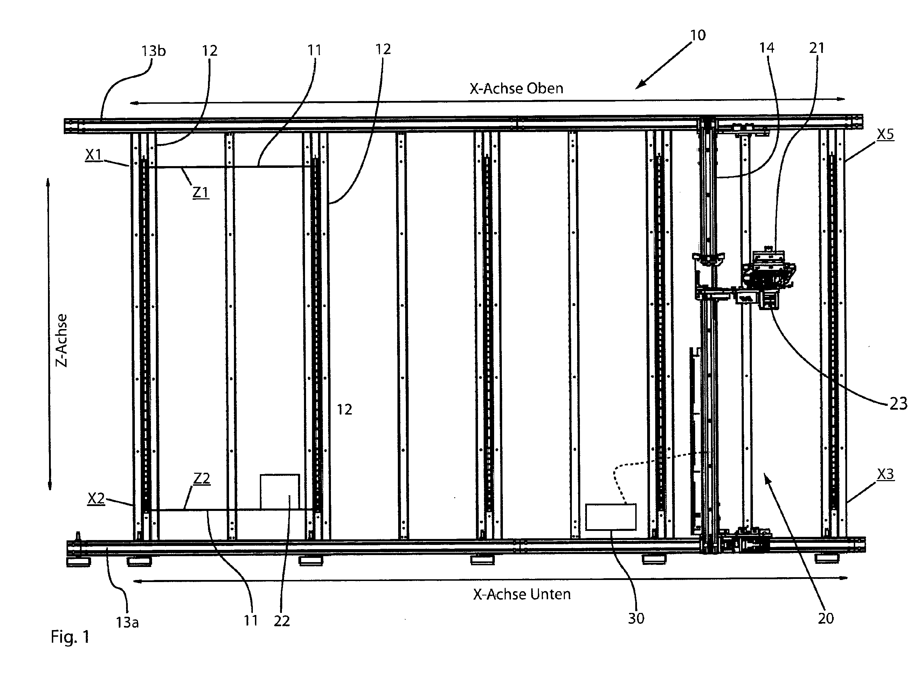

Figur 1 zeigt eine seitliche Schnittansicht einer Apothekenkommissioniervorrichtung mit zwei Regalreihen, von denen jedoch lediglich eine Regalreihe 10 zu erkennen ist. Die Apothekenkommissioniervorrichtung umfasst ferner ein zwischen den Regalreihen 10 horizontal und vertikal verfahrbares Bediengerät 20. Die Regalreihen umfassen jeweils eine Mehrzahl von sich in horizontaler Richtung (X-Achse) erstreckender Regalböden 11 und eine Mehrzahl von sich in vertikaler Richtung (Z-Achse) erstreckender Regalwänden 12. Üblicherweise sind die Regalböden 11 vollständig aus Glas mit einer glatten Oberfläche gefertigt. Auf den Regalböden 11 werden in chaotischer Weise bei optimaler Raumausnutzung Arzneimittelpackungen 22 gelagert.

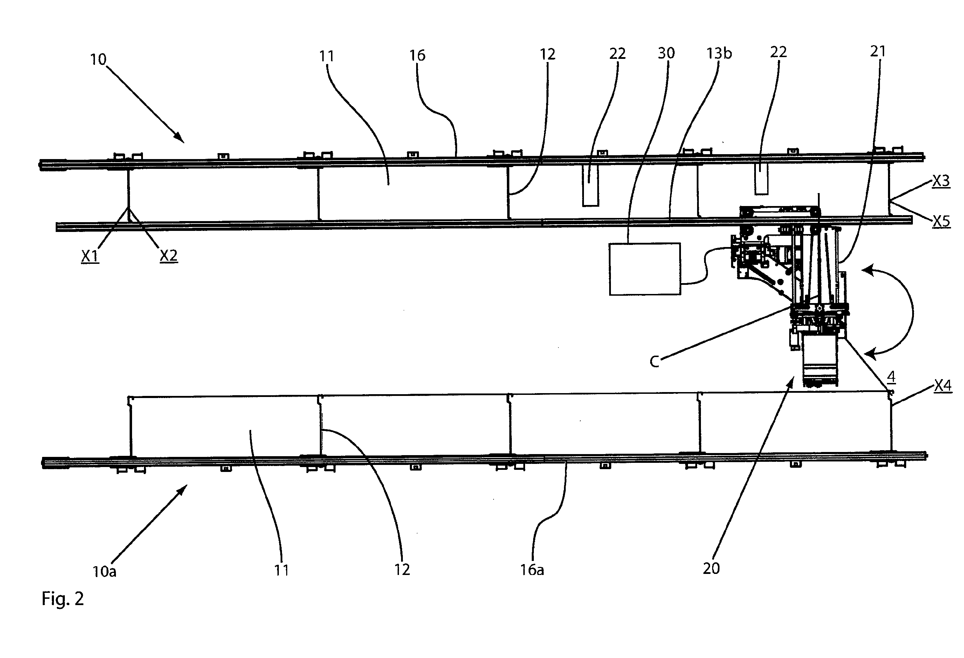

Das Bediengerät 20 ist mit Hilfe von zwei horizontalen und einer vertikalen Führungsschiene (13a, 13b, 14) und diesen zugeordneten Antriebseinrichtungen in horizontaler und vertikaler Richtung zwischen den Regalreihen 10 verfahrbar. Die vertikale Führungsschiene 14 ist zu diesem Zweck verfahrbar an den horizontalen Führungsschienen 13a, 13b befestigt. Das Bediengerät 20 umfasst eine Greifvorrichtung 21, die über eine entsprechende Antriebseinrichtung vertikal an der Führungsschiene 14 verfahrbar ist, sowie einen Backen- und/oder Sauggreifer. Die Greifvorrichtung 21 umfasst ferner einen Sensor 23, mit welchem der Abstand von dem Sensor zu der Rückwand (siehe Figur 2) der Regalreihe, eingelagerten Arzneimittelpackungen 22 oder Bauteilen einer Regalreihe (Regalwände, Regalböden) ermittelt werden kann.

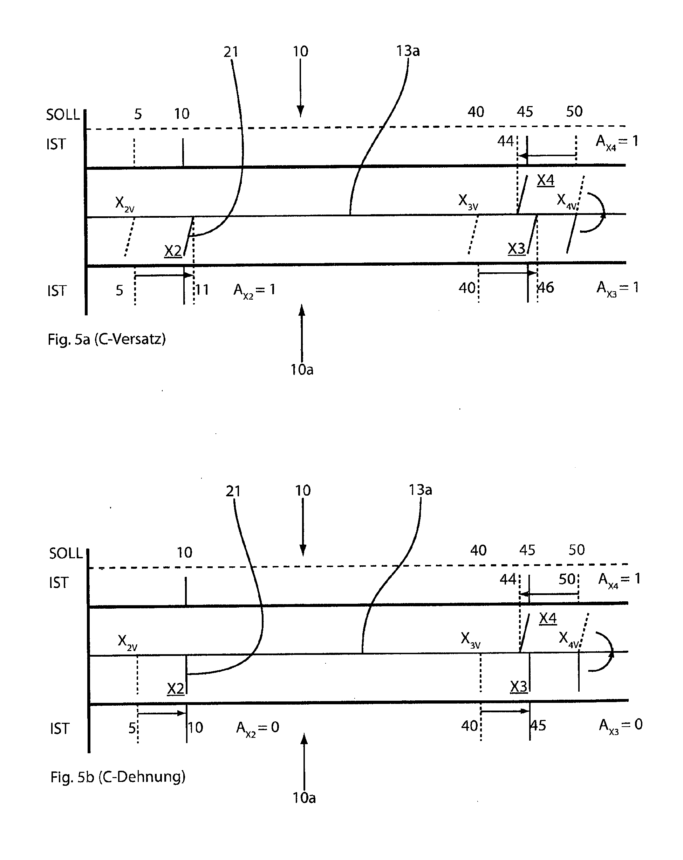

Bei dem Sensor 23 kann es sich beispielsweise um einen Optischen Sensor nach dem Triangulationsprinzip handeln, der den Abstand in einem 90°-Winkel zu der durch die beiden Horizontalführungen aufgespannten Ebene ermittelt (Idealwert, Positionierungsabweichungen möglich, siehe dazu Figuren 5a, 5b). Bei einer alternativen Ausführungsform kann auch ein induktiver Näherungssensor verwendet werden, wobei in diesem Fall metallische Referenzpunkte zu verwenden sind.

Das Bediengerät 20 ist elektronisch mit einer lediglich schematisch dargestellten Steuereinrichtung 30 gekoppelt. Die Steuereinrichtung 30 kann mehrere (nicht dargestellte) Computer umfassen und steuert den gesamten Betrieb der Anlage (Identifizieren, Einlagern, Auslagern etc.).

Bei der in Figur 1 gezeigten Apothekenkommissioniervorrichtung sind sieben Referenzpositionen (X1, X2, X3, X4, X5, Z1, Z2) vorgesehen (Referenzposition X4 ist an der nicht dargestellten Regalreihe angeordnet und daher in Figur 1 nicht "sichtbar"). Diese Anzahl von Referenzpositionen ist jedoch nur dann notwendig, wenn sämtliche der nachfolgend beschriebenen Positionierungsabweichungen ermittelt werden sollen - bei anderen Ausführungsformen kann lediglich eine Referenzposition ausreichend sein.

Referenzpositionen können durch beliebige von dem Sensor erfassbare Punkte innerhalb der Apothekenkommissioniervorrichtung bereitgestellt werden. Bei der nachfolgenden Beschreibung des Verfahrens wird davon ausgegangen, dass die Referenzpositionen von Regalböden (Positionen Z1, Z2)und Regalwänden (Positionen X1, X2, X3, X4, X5) bereitgestellt werden; die Referenzpositionen sind in diesem Falle also keine zusätzlichen baulichen Maßnahmen. Bei anderen Ausführungsformen können die Referenzpositionen auch durch spezielle Bauteile (Signalgeber etc.) bereitgestellt werden.

Figur 2 zeigt eine Draufsicht auf einen Abschnitt einer Apothekenkommissioniervorrichtung, wobei in dieser Figur die beiden parallelen Regalreihen 10, 10a ersichtlich sind, zwischen denen das Bediengerät 20 mit Hilfe der Führungsschienen 13a, 13b, 14 horizontal und vertikal verfahrbar ist. Zur Ein- oder Auslagerung von Arzneimittelpackungen ist die Greifvorrichtung 21 des Bediengerätes 20 in einem 90°-Winkel zu der Rückwand 16, 16a der entsprechenden Regalreihe 10, 10a ausgerichtet. Da das Bediengerät 20 selber wartungs- und kostenintensiv ist, ist auch bei einer Apothekenkommissioniervorrichtung mit zwei parallelen Regalreihen üblicherweise ein Bediengerät verbaut. Um die beiden Regalreihen bedienen zu können, ist die Greifvorrichtung um eine Drehachse C drehbar, wie dies in Figur 2 angedeutet ist. Von einem Bauteil der Regalreihe 10a (Regalwand 12) wird die in Figur 1 nicht "sichtbare" Referenzposition X4 bereitgestellt.

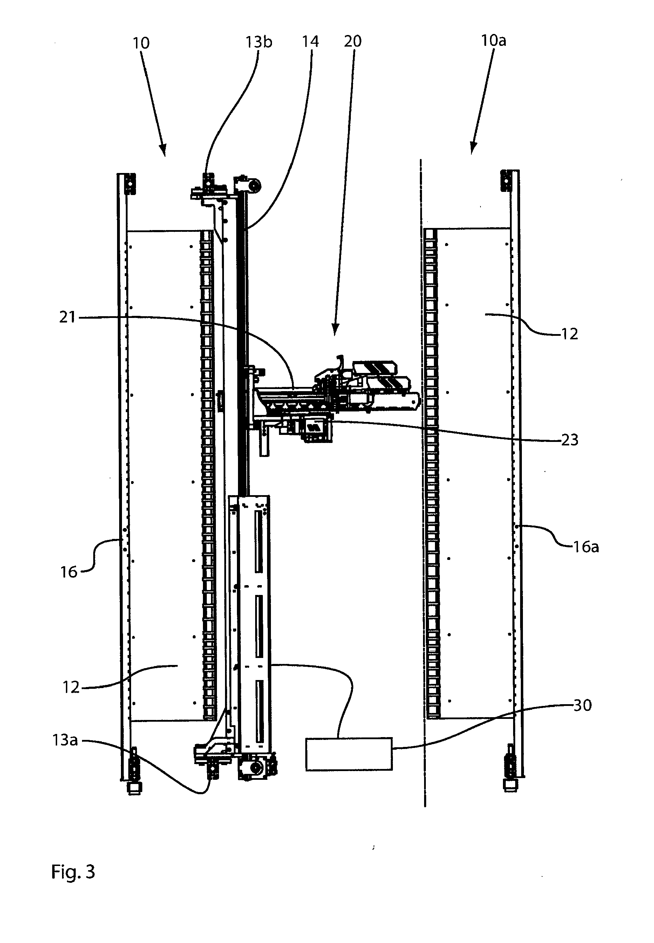

Figur 3 zeigt eine Schnittansicht der Apothekenkommissioniervorrichtung. Zwischen den zwei Regalreihen 10, 10a ist das Bediengerät 20 auf Führungsschienen 13a, 13b horizontal und vertikal verfahrbar. Details dazu, wie das Bediengerät an den Führungsschienen verfahrbar ist, sind dem Fachmann bekannt und für die vorliegende Erfindung nicht wesentlich. Üblicherweise wird die vertikale Führungsschiene 14 mit Hilfe von einem oder zwei Zahnriemen und einem oder mehreren Antrieben an den horizontalen Führungsschienen 13a, 13b verfahren. Ebenso wird üblicherweise die Greifvorrichtung 21 mit dem Sensor 23 mit Hilfe eines Zahnriemens und eines entsprechenden Antriebs vertikal an der vertikalen Führungsschiene 14 verfahren.

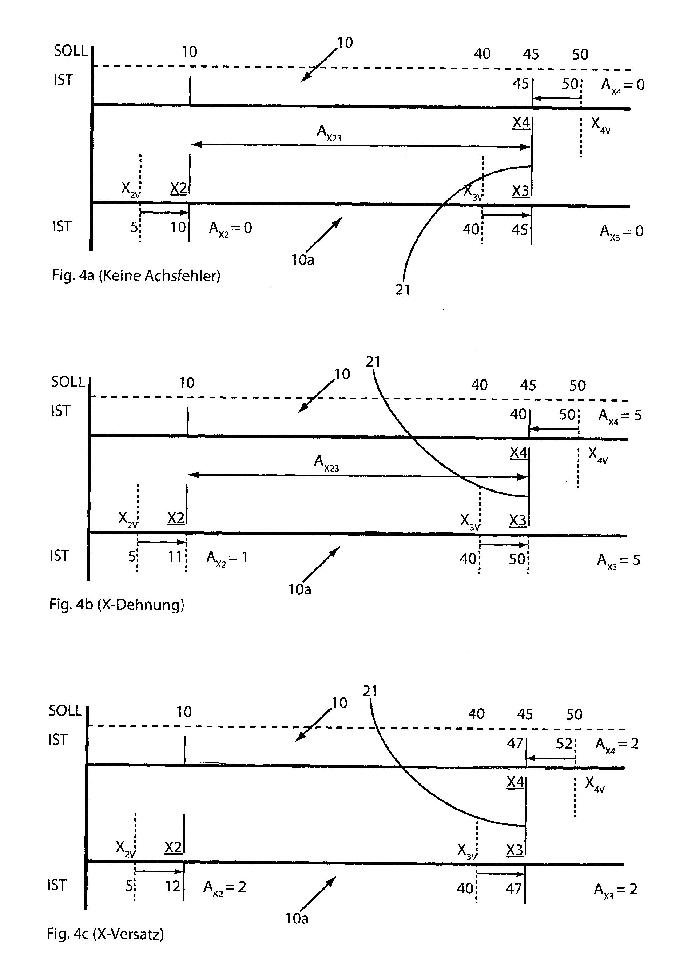

Unter Bezugnahme auf die Figuren 4a - 4c, 5a - 5b und 6 werden nachfolgend Ausführungsformen des erfindungsgemäßen Verfahrens beschrieben. Die für die Veranschaulichung des erfindungsgemäßen Verfahrens gewählten Darstellungen von Abschnitten einer Apothekenkommissioniervorrichtung sind sehr schematisch, um das Wesen des erfindungsgemäßen Verfahrens nicht durch unnötige bauliche Details zu verschleiern.

### Positionierungsabweichung X-Achse

Anhand der Figuren 4a - 4c wird nachfolgend eine Ausführungsform des erfindungsgemäßen Verfahrens beschrieben, wobei diese Ausführungsform auf Positionierungsabweichungen hinsichtlich der X-Achse beschränkt ist.

Eine Positionierungsabweichung hinsichtlich der X-Achse lässt sich generell anhand eines Referenzpunktes (bezogen auf einen Bezugspunkt) feststellen; anhand dieser einen ermittelten Abweichung lässt sich aber keine Aussage über die Art der Abweichung machen (Längendehnung Zahnriemen, mechanischer Schlupf etc.). Im nachfolgenden wird daher eine Ausführungsform des erfindungsgemäßen Verfahrens beschrieben, bei dem zwei Referenzpunkte (X2, X3) zur Ermittlung einer Positionierungsabweichung verifiziert werden.

Die Figuren 4a - 4c zeigen eine schematische Schnittansicht eines Abschnitts einer Apothekenkommissioniervorrichtung mit zwei Regalreihen 10, 10a und einem zwischen den Regelreihen horizontal und vertikal verfahrbaren Bediengerät, von welchen der Übersichtlichkeit halber lediglich die Greifvorrichtung 21 schematisch angedeutet ist. Die Ansicht gemäß den vorgenannten Figuren zeigt den unteren Teil der Apothekenkommissioniervorrichtung,und daher sind lediglich die Referenzpositionen X2, X3*,* X4 angedeutet.

Die in den Figuren gemachten Zahlenangaben geben den X-Anteil von Raumkoordinaten verschiedener Referenzpositionen wieder, wobei die verwendeten Zahlen das Verfahren als solches lediglich veranschaulichen sollen.

Bei dem erfindungsgemäßen Verfahren zum Betreiben einer Apothekenkommissioniervorrichtung werden zum Erkennen einer Positionierungsabweichung des Bediengerätes in horizontaler Richtung (X-Achse) zwei Sollwerte X_{2S}, X_{3S} für die Referenzpositionen X2, X3 von Regalreihe 10a bereitgestellt. Die Sollwerte der vorgenannten Referenzpositionen sind bei den Figuren 4a - 4c jeweils in der links mit "SOLL" bezeichneten Reihe aufgeführt (X_{2S} = 10, X_{3S} = 45).

Wie bereits erwähnt, können die vorgenannten Sollwerte bereitgestellt werden, indem diese lediglich in dem Speicher der Steuereinrichtung abgelegt werden, oder indem diese beispielsweise bei einer (Erst)Inbetriebnahme der Apothekenkommissioniervorrichtung erlernt werden. Bei der (Erst)Inbetriebnahme wird dazu das Bediengerät von einer Bezugsposition, deren Position vorgegeben ist (z.B. "Nullpunkt" des Bediengeräts), mit einer ersten Geschwindigkeit (vorzugsweise Maximalgeschwindigkeit des Bediengerätes in X-Richtung) zu einer dem ersten Sollwert X_{2S} entsprechenden Vorposition X_{2V} verfahren. Ab dieser Vorposition X_{2V} wird das Bediengerät mit eingeschaltetem Sensor in Richtung auf die Referenzposition X2 weiterbewegt. Sobald die Referenzposition erreicht ist, detektiert der Sensor ein charakteristisches Signal. In dem gezeigten Ausführungsbeispiel wird die Referenzposition X2 durch eine Regalwand bereitgestellt, und der Sensor erfasst ein charakteristisches Signal (Flankenwechsel bei digitalem Ausgangssignal oder Pegelwechsel bei analogem Ausgabesignal). Die Raumkoordinaten (oder zumindest der X-Anteil dieser) wird als Sollwert X_{2S} für die Referenzposition X2 abgespeichert. Entsprechend wird mit der Referenzposition X3 verfahren, wobei ein Rückfahren des Bediengerätes zu dem Bezugspunkt unterbleiben kann. Gemäß der Figur 4a werden die Referenzpositionen X2 und X3 von links angefahren. Dies ist jedoch nicht erforderlich; von welcher Seite die Referenzpositionen mit eingeschaltetem Sensor angefahren werden, ist für das Verfahren unerheblich.

Nach der Bereitstellung der Sollwerte für die Referenzposition X2, X3 wird mit dem Bediengerät 20 eine dem Sollwert X_{2S} entsprechende Position in horizontaler Richtung angefahren. Die vorgenannte Position X_{2V} befindet sich bei den X-Koordinaten 5. Mit angeschaltetem Sensor wird nun das Bediengerät weiter auf den Sollwert zu bewegt und bei Erfassen eines für die Referenzposition X2 charakteristischen Signals wird ein Istwert X_{2I} für die Referenzposition X2 ermittelt. Sobald der Istwert für die Referenzposition X2 ermittelt wurde, wird das Bediengerät an eine dem Sollwert X3 entsprechende Position X_{3V} verfahren und ab dieser Position wird das Bediengerät 20 weiter in Richtung auf die Referenzposition X3 bewegt, und bei Erfassen eines für die Referenzposition X3 charakteristischen Signals wird ein Istwert X_{3I} für diese Referenzposition ermittelt.

Die ermittelten Istwerte X_{2I}, X_{3I} werden mit den entsprechenden Sollwerten X_{2S}, X_{3S} verglichen und eine Abweichung pro Referenzposition X2 und X3 ermittelt (A_{X2}, A_{X3}). Eine Abweichung kann ferner bestimmt werden, in dem die beiden ermittelten Istwerte für die Referenzpositionen X2, X3 voneinander abgezogen werden (also der Abstand zwischen den Istwerten X_{2I}, X_{3I} ermittelt wird) und der ermittelte Wert mit der Differenz der entsprechenden Sollwerte verglichen wird.

Sofern eine einem Grenzwert überschreitende Abweichung A_{X2}, A_{X3}, A_{X23} ermitteln wird, wird ein auf die Notwendigkeit einer Korrektur hinweisendes Signal ausgegeben. Beispielsweise kann dem Benutzer angezeigt werden, dass eine Positionierungsabweichung bezüglich der X-Achse ermittelt wurde und der Service entsprechend informiert wurde. Sofern die ermittelten Abweichungen einen sicheren Betrieb der Anlage nicht mehr zulassen, wird diese gestoppt und der Service informiert.

Die ermittelten Abweichungen lassen Rückschlüsse auf die Art der Störung der Positionierungsgenauigkeit zu. In dem in Figur 4a gezeigten Fall stimmen die Istwerte für die Referenzpositionen X2, X3 mit den Sollwerten überein; daraus folgt, dass keine Störung der Positionierungsgenauigkeit hinsichtlich der X-Achse vorliegt. Bei dem in Figur 4b gezeigten Fall wird hinsichtlich der Referenzposition X2 eine Abweichung A_{X2} = 1 und hinsichtlich der Referenzposition X3 eine Abweichung A_{X3} = 5 ermittelt, woraus geschlossen werden kann, dass eine Längendehnung des Zahnriemens bzw. der Zahnriemen der Antriebseinrichtung für die X-Achse vorliegt. Anhand der ermittelten Abweichungen kann ein Korrekturfaktor ermittelt werden, der bei der weiteren Positionierung des Bediengerätes verwendet werden kann.

Bei dem in Figur 4c gezeigten Fall wird für die beiden Referenzpositionen X2, X3 jeweils eine Abweichung A_{X2}, A_{X3} = 2 ermittelt. Daraus folgt, dass keine Längendehnung des bzw. der Zahnriemen der Antriebseinrichtung für die Bewegung des Bediengerätes in X-Richtung vorliegt, jedoch ein Versatz, der auf einen mechanischen Schlupf oder ein Springen des Zahnriemens über ein entsprechendes Antriebszahnrad hinweist.

### Positionierungsabweichung C-Achse (Drehachse Greifvorrichtung Bediengerät)

Anhand der Figuren 4a, 5a, 5b wird nachfolgend eine Ausführungsform des erfindungsgemäßen Verfahrens beschrieben, bei welchem die Positionierungsgenauigkeit hinsichtlich der C-Achse (Drehachse der Greifvorrichtung des Bediengeräts) ermittelt wird.

Bei dieser Ausführungsform des erfindungsgemäßen Verfahrens werden zunächst die Sollwerte für die Referenzpositionen X3, X4 wie bereits beschrieben bereitgestellt. Anschließend wird auf die bereits unter Bezugnahme auf die X-Achse beschriebene Weise die Positionierungsabweichung hinsichtlich der Referenzposition X3 ermittelt. Nachfolgend wird eine Positionsabweichung hinsichtlich der X-Achse bei einer Referenzposition X4 der anderen Regalreihe 10 ermittelt.

Dazu wird die Greifvorrichtung 21 des Bediengerätes um einen vorgegebenen Wert C gedreht, wobei diese Drehung um den vorgegebenen Wert C im Idealfall einer Drehung um 180° entspricht. Dann wird mit dem Bediengerät eine zweite, einem zweiten Sollwert X_{4S} entsprechende Position X_{4V} angefahren. Bei dem in den Figuren 4a, 5a - 5b angezeigten schematischen Darstellungen liegt diese Position X_{4V} rechts von der Referenzposition X4. Bei eingeschaltetem Sensor wird anschließend das Bediengerät weiter in Richtung auf die Referenzposition X4 verfahren und bei Erfassen eines für die Referenzposition X4 charakteristischen Signals ein Istwert X_{4I} der Referenzposition ermittelt. Anschließend wird der Sollwert der zweiten Referenzposition X4 mit dem Istwert verglichen und eine Abweichung A_{X4} bestimmt. Sofern die für die Referenzpositionen X3 und X4 ermittelten Abweichungen Grenzwerte überschreiten oder sich um einen vorgegebenen Wert unterscheiden, wird ein die Notwendigkeit einer korrekturanzeigendes Signal ausgegeben.

Aus den ermittelten Abweichungen A_{X3}, A_{X4} lassen sich Rückschlüsse auf die Art der Positionierungsabweichung gewinnen. Bei dem in Figur 4a gezeigten Fall stimmen die Abweichungen für die Referenzpositionen X3 und X4 überein (sie sind beide 0), wobei dies bedeutet, dass keine Positionierungsabweichung hinsichtlich der C-Drehachse vorliegt (sofern andere Positionierungsabweichungen hinsichtlich der X-Achse ausgeschlossen werden können).

Figur 5a zeigt einen Fall, bei welchem die Greifvorrichtung 21 nicht in einem 90°-Winkel zu der Regalreihe 10a orientiert ist, sondern der Winkel zu der Regalreihe geringfügig im Uhrzeigersinn verstellt ist. Bei dem Ermitteln des Istwertes für den Referenzposition X3 wird dieser daher nicht (wie dies bei einem 90°-Winkel der Fall wäre) bei X = 45 detektiert, sondern erst bei X = 46. Nach Drehung der Greifvorrichtung 21 um einen Wert C (der in diesem Fall dem Idealwinkel von 180° entspricht) wird der Istwert für die Referenzposition X4 ermittelt. Aufgrund der Fehlstellung der Greifvorrichtung in Bezug auf die Regalreihe 10 wird der Istwert der Referenzposition bei X = 44 ermittelt. Eine Vergleich der Soll- mit den Istwerten für die Referenzpositionen X3, X4 zeigt, dass die Abweichungen gleich sind (A_{X3}, A_{X4}) und größer 0, was auf eine Fehlstellung der Greifvorrichtung 21 in Bezug auf die Regalwände schließen lässt.

Figur b5 zeigt einen Fall, in dem die Drehung um den Betrag C keine Drehung um 180°, sondern um einen verminderten Winkel bedingt. Hinsichtlich der Referenzposition X3 wird eine Abweichung von A_{X3} = 0 ermittelt, für die Referenzposition X4 eine Abweichung A_{X4} = 1. Bei Annahme einer fehlerfreien Positionierung hinsichtlich der X-Achse bedeutet dies, dass die Drehung um einen Wert C keine Drehung um 180° bewirkt, die Positionierungsgenauigkeit hinsichtlich der C-Achse also gestört ist.

### Positionierungsabweichung Z-Achse

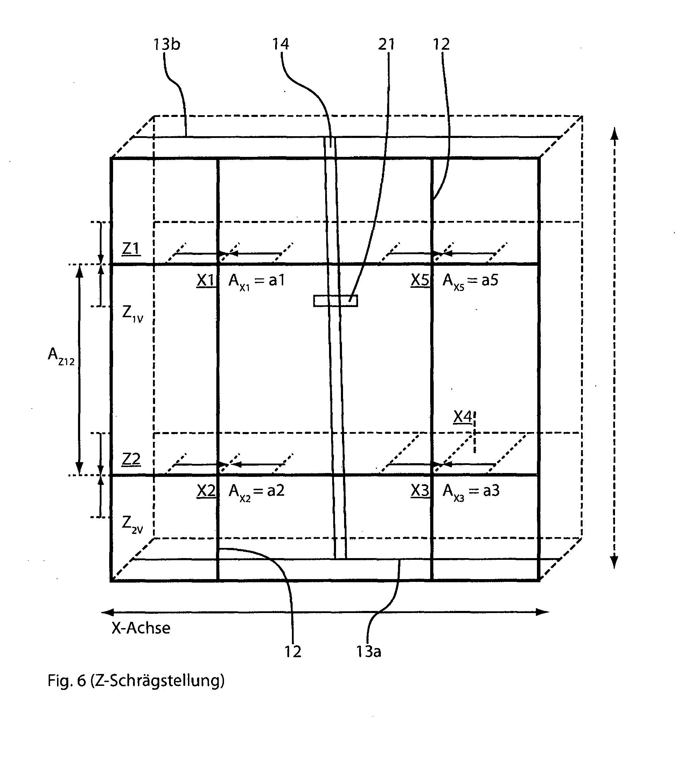

Anhand der Figur 6 wird anschließend kurz eine Ausführungsform des erfindungsgemäßen Verfahrens beschrieben, bei welchem zusätzlich eine Positionierungsabweichung hinsichtlich der Z-Achse ermittelt wird.

Eine Positionierungsabweichung hinsichtlich der Z-Achse lässt sich generell entsprechend der Positionierungsabweichung hinsichtlich der X-Achse ermitteln, d. h. es wird entweder eine Positionierungsabweichung, ausgehend von einem Bezugspunkt, hinsichtlich der Referenzpositionen Z1, Z2 ermitteln, oder es wird eine Differenz der Istwerte der Bezugspunkte Z1, Z2 ermittelt und diese mit der Differenz zwischen den Sollwerten der Bezugspunkte Z1 und/oder Z2 verglichen. Wird eine Abweichung zwischen der Differenz der Soll- und der Istwerte erkannt, kann man daraus auf eine Längendehnung eines ggf. verwendeten Zahnriemens zur Positionierung der Greifvorrichtung 21 schließen. Die Details hinsichtlich der Durchführung des Verfahrens zur Bestimmung einer Positionierungsabweichung der Z-Achse sind mit denen des Verfahrens zum Ermitteln einer Positionierungsabweichung hinsichtlich der X-Achse vergleichbar, so dass hier auf eine erneute detaillierte Darstellung verzichtet wird.

Um eine etwaige Schrägstellung der vertikalen Führungsschiene zu ermitteln werden Positionierungsabweichungen bei zwei Referenzpositionen mit unterschiedlichen Z-Achsen-Positionen durchgeführt. Im vorliegenden Fall kann eine mögliche Schrägstellung der Z-Achse ermittelt werden, in den Positionierungsabweichungen hinsichtlich der X-Achse für die Referenzpositionen X1, X2 oder X3, X5 ermittelt werden. Bei der Ermittlung einer möglichen Schrägstellung der Z-Achse sollten die beiden vermessenen Referenzpositionen an einer Regalreihe angeordnet sein, um mögliche Einflüsse durch eine Positionierungsabweichung hinsichtlich der C-Drehachse der Greifvorrichtung auszuschließen. Die möglichen Positionierungsabweichungen hinsichtlich der beiden Referenzpositionen werden verglichen und bei einer Abweichung dieser voneinander kann auf eine Schrägstellung der Z-Achse (vertikale Führungsschiene) geschlossen werden, und bei Überschreitung eines Grenzwertes wird ein die Notwendigkeit einer korrekturhinweisendes Signal ausgegeben.

## Patentansprüche

1. Verfahren zum Betreiben einer Apothekenkommissioniervorrichtung mit
zumindest einer Regalreihe (10) mit jeweils einer Mehrzahl von sich in einer horizontalen Richtung (X-Achse) erstreckenden Regalböden (11) und einer Mehrzahl von sich in einer vertikalen Richtung (Z-Achse) ersteckenden Regalwänden (12),
zumindest einem, vor der Regalreihe (10) horizontal und vertikal verfahrbaren Bediengerät (20) mit einer Greifvorrichtung (21) zum Ein- und/oder Auslagern von Arzneimittelpackungen (22) auf die bzw. von den Regalböden (11), wobei das Bediengerät zumindest einen Sensor (23) aufweist, und
einer mit dem Bediengerät (20) gekoppelten Steuereinheit (30),
wobei zum Erkennen einer Positionierungsabweichung des Bediengerätes in horizontaler Richtung (X-Achse)
a) zumindest ein Sollwert (X_{1S}, X_{2S}, X_{3S}, X_{4S}, X_{5S}) zumindest einer Referenzposition bereitgestellt wird
b) mit dem Bediengerät (20) eine einem Sollwert entsprechende Position in horizontaler Richtung angefahren wird,
c) bei Erfassen eines für eine Referenzposition charakteristischen Signals ein Istwert (X_{1I}, X_{2I}, X_{3I}, X_{4I}, X_{5I}) dieser Referenzposition ermittelt wird,
d) ein Sollwert (X_{1S}, X_{2S}, X_{3S}, X_{4S,} X_{5S}) mit einem entsprechenden Istwert (X_{1I}, X_{2I}, X_{3I}, X_{4I}, X_{5I}) oder zwei Istwerte miteinander verglichen und eine Abweichung (A_{X1}, A_{X2}, A_{X3}, A_{X4}, A_{X15}, A_{X23}) bestimmt wird, und,
e) sofern eine einen Grenzwert überschreitende Abweichung ermittelt wird, ein auf die Notwendigkeit einer Korrektur hinweisendes Signal ausgegeben wird.

2. Verfahren zum Betreiben einer Apothekenkommissioniervorrichtung nach Anspruch 1, wobei zum Erkennen einer Positionierungsabweichung des Bediengerätes in vertikaler Richtung (Z-Achse)
a) zumindest ein Sollwert (Z_{1S}, Z_{2S}) zumindest einer Referenzposition bereitgestellt wird
b) mit dem Bediengerät (20) eine einem Sollwert entsprechende Position angefahren wird,
c) bei Erfassen eines für eine Referenzposition charakteristischen Signals ein Istwert (Z_{1I}, Z_{2I}) dieser Referenzposition ermittelt wird,
d) ein Sollwert (Z_{1S}, Z_{2S}) mit einem entsprechenden Istwert (Z_{1I}, Z_{2I}) oder zwei Istwerte miteinander verglichen und eine Abweichung (A_{Z1}, A_{Z2}, A_{Z12}) bestimmt wird, und,
e) sofern eine einen Grenzwert überschreitende Abweichung ermittelt wird, ein auf die Notwendigkeit einer Korrektur hinweisendes Signal ausgegeben wird.

3. Verfahren zum Betreiben einer Apothekenkommissioniervorrichtung nach Anspruch 1 oder 2, wobei
die Apothekenkommissioniervorrichtung zumindest zwei parallele Regalreihen (10, 10a) aufweist, zwischen denen das Bediengerät (20) horizontal und vertikal verfahrbar ist und wobei die Greifvorrichtung (21) des Bediengerätes um die Vertikalachse (C-Drehachse) schwenkbar ist,
wobei zum Erkennen einer Positionierungsabweichung der Greifvorrichtung (21) bezüglich der C-Drehachse nach dem Erkennen einer Positionierungsabweichung des Bediengerätes in horizontaler Richtung (X-Achse) bei einer der Regalreihen (10, 10a) [Schritte a) - e) aus Anspruch 1) die Greifvorrichtung um einen vorgegebenen Wert C gedreht wird und
a) mit dem Bediengerät (20) eine zweite, einem zweiten Sollwert (X_{4S}) entsprechende Position angefahren wird,
b) bei Erfassen eines für die zweite Referenzposition charakteristischen Signals ein Istwert (X_{4I}) der Referenzposition ermittelt wird,
c) der Sollwert der zweiten Referenzposition (X_{4S}) mit dem Istwert (X_{4I}) verglichen und eine Abweichung (A_{X4}) bestimmt wird, und,
d) sofern die ermittelten Abweichungen (AX3, AX4) hinsichtlich der Positionierung in X-Richtung bei der ersten und der zweiten Regalreihe (A_{X3}, A_{X4}) Grenzwerte überschreiten oder sich um einen vorgegebenen Wert unterscheiden, ein die Notwendigkeit einer Korrektur anzeigendes Signal ausgegeben wird.

4. Verfahren zum Betreiben einer Apothekenkommissioniervorrichtung nach einem der vorgenannten Ansprüche, wobei die Apothekenkommissioniervorrichtung zwei horizontale Führungsschienen (13a, 13b) und eine vertikale Führungsschiene (14) umfasst, wobei die vertikale Führungsschiene entlang der horizontalen Führungsschienen und die Greifvorrichtung entlang der vertikalen Führungsschiene (14) verfahrbar ist,
wobei zum Erkennen einer Schrägstellung des Bediengerätes
a) für die untere und die obere Führungsschiene (13a, 13b) zumindest ein Sollwert (X_{1S}, X_{2S}) zumindest einer Referenzposition bereitgestellt wird
b) mit dem Bediengerät (20) eine den Sollwerten (X_{3S}, X_{4S}) entsprechende Position angefahren wird,
c) bei Erfassen eines für eine Referenzposition charakteristischen Signals ein Istwert (X_{3I}, X_{4I}) der Referenzposition ermittelt wird,
d) die Sollwerte (X_{3S}, X_{4S}) den Istwerten (X_{3I}, X_{4I}) verglichen und eine Abweichungen (A_{X3}, A_{X4}) pro Referenzposition bestimmt wird, und,
e) sofern sich die ermittelten Abweichungen (A_{X3}, A_{X4}) selbst um einen vorgegebenen Wert unterscheiden, ein die Notwendigkeit einer Korrektur anzeigendes Signal ausgegeben wird.

5. Verfahren zum Betreiben einer Apothekenkommissioniervorrichtung nach einem der vorgenannten Ansprüche, wobei in Abhängigkeit von dem die Notwendigkeit einer Korrektur anzeigenden Signal
eine Serviceanfrage elektronisch an einen Servicemitarbeiter übermittelt wird, und/oder
die Apothekenkommissioniervorrichtung (1) stillgesetzt wird.

6. Verfahren zum Betreiben einer Apothekenkommissioniervorrichtung nach einem der Ansprüche 1 - 5, wobei der Sollwert bzw. die Sollwerte (X_{1S}, X_{2S}, X_{3S}, X_{4S}, X_{5S}, X_{5S}, Z_{1S}, Z_{2S}) zumindest einer Referenzposition bereitgestellt wird bzw. werden, indem der Sollwert bzw. die Sollwerte in der Steuereinrichtung (X) als vorgegebene Werte hinterlegt werden.

7. Verfahren zum Betreiben einer Apothekenkommissioniervorrichtung nach einem der Ansprüche 1 - 5, wobei der Sollwert bzw. die Sollwerte (X_{1S}, X_{2S}, X_{3S}, X_{4S}, X_{5S}, Z_{1S}, Z_{2S}) zumindest einer Referenzposition bereitgestellt wird bzw. werden, indem der Sollwert bzw. die Sollwerte nach der ersten Inbetriebnahme der Apothekenkommissioniervorrichtung erlernt werden, indem vorgegebene Referenzpositionen angefahren werden und bei Erfassen eines für eine bestimmte Referenzposition charakteristischen Signals ein Wert ermittelt, aus diesem ein Sollwert ermittelt und in der Steuereinrichtung (XX) gespeichert wird.

8. Verfahren zum Betreiben einer Apothekenkommissioniervorrichtung nach einem der Ansprüche 1 - 5, wobei die einem Sollwert (X_{1S}, X_{2S}, X_{3S}, X_{4S}, X_{5S}, Z_{1S}, Z_{2S}) entsprechende Position mit einer ersten Geschwindigkeit v1 angefahren wird, das Bediengerät (20) mit einer zweiten Geschwindigkeit v2 weiter in Richtung auf die Referenzposition verfahren wird, bis ein für die Referenzposition charakteristischen Signal erfasst wird.

9. Verfahren zum Betreiben einer Apothekenkommissioniervorrichtung nach einem der Ansprüche 1 - 8, **dadurch gekennzeichnet, dass** auf der Basis des oder der ermittelten Abweichung(en) ein oder mehrere entsprechende Korrekturfaktoren ermittelt werden, diese in der Steuereinrichtung gespeichert werden und nachfolgend ausgeführte Steuerprogramme bei dem Ansteuern des Bediengerätes den bzw. die Korrekturfaktoren berücksichtigen.

## Claims

1. A method for operating a pharmacy picking device with
at least one row of shelves (10) each with a plurality of shelf floors (11) extending in a horizontal direction (X-axis) and a plurality of shelf walls (12) extending in vertical direction (Z-axis),
at least one operator unit (20) horizontally and vertically movable in front of the row of shelves (10) with a gripping device (21) for storing and/or retrieving medicine packages (22) on or from the shelf floors (11), wherein the operator unit comprises at least one sensor (23), and
a control unit (30) coupled with the operator unit (20),
wherein for detecting a positional deviation of the operator unit in horizontal direction (X-axis)
a) at least one set value (X_{1S}, X_{2S}, X_{3S}, X_{4S}, X_{5S}) of at least one reference position is provided,
b) the operator unit (20) is moved to a position corresponding to the set value in horizontal direction,
c) when a signal characteristic of the reference position is detected, an actual value (X_{1I}, X_{2I}, X_{3I}, X_{4I}, X_{5I}) of this reference position is ascertained,
d) a set value (X_{1S}, X_{2S}, X_{3S}, X_{4S}, X_{5S}) is compared with the corresponding actual value (X_{1I}, X_{2I}, X_{3I}, X_{4I}, X_{5I}) or two actual values are compared with each other and a deviation (A_{X1}, A_{X2}, A_{X3}, A_{X4}, A_{X15}, A_{X23}) is determined, and,
e) insofar as a deviation exceeding a threshold value is ascertained, a signal is emitted indicating that a correction is required.

2. The method for operating a pharmacy picking device according to claim 1, wherein for detecting a positional deviation of the operator unit in vertical direction (Z-axis)
a) at least one set value (Z_{1S}, Z_{2S}) of at least one reference position is provided,
b) the operator unit (20) is moved to a position corresponding to the set value,
c) when a signal characteristic of the reference position is detected, an actual value (Z_{1I}, Z_{2I}) of this reference position is ascertained,
d) a set value (Z_{1S}, Z_{2S}) is compared with the corresponding actual value (2_{1I}, Z_{2I}) or two actual values are compared with each other and a deviation (A_{Z1}, A_{Z2}, A_{Z12}) is determined, and
e) insofar as a deviation exceeding a threshold value is ascertained, a signal is emitted indicating that a correction is required.

3. The method for operating a pharmacy picking device according to claim 1 or 2, wherein
the pharmacy picking device comprises at least two parallel rows of shelves (10, 10a) between which the operator unit (20) is horizontally and vertically movable and wherein the gripping device (21) of the operator unit can be swivelled about the vertical axis (C-rotary axis),
wherein for detecting a positional deviation of the gripping device (21) with respect to the C-rotary axis following detection of a positional deviation of the operator unit in horizontal direction (X-axis), the gripping device is rotated by a specified value C at one of the rows of shelves (10, 10a) [steps a) - e) in claim 1], and
a) the operator unit (20) is moved to a second position corresponding to a second set value (X_{4S}),
b) when a signal characteristic of the second reference position is detected, an actual value (X_{4I}) of the reference position is ascertained,
c) the set value of the second reference position (X_{4S}) is compared with the actual value (X_{4I}) and a deviation (A_{X4}) is determined, and
d) insofar as the ascertained deviations (AX3, AX4) with respect to the positioning in X-direction at the first and the second row of shelves (A_{X3}, A_{X4}) exceed threshold values or are different from the specified value, a signal is issued indicating that a correction is required.

4. The method for operating a pharmacy picking device according to one of the preceding claims, wherein the pharmacy picking device comprises two horizontal guide rails (13a, 13b) and one vertical guide rail (14), wherein the vertical guide rail is movable along the horizontal guide rail and the gripping device is movable along the vertical guide rail (14),
wherein for detecting an oblique position of the operator unit
a) at least one set value (X_{1S}, X_{2S}) of at least one reference position is provided for the lower and the upper guide rail (13a, 13b),
b) the operator unit (20) is moved to a position corresponding to the set values (X_{3S}, X_{4S}),
c) when a signal characteristic of the reference position is detected, an actual value (X_{3I}, X_{4I}) of the reference position is ascertained,
d) the set values (X_{3S}, X_{4S}) are compared with the actual value (X_{3I}, X_{4I}) and a deviation (A_{X3}, A_{X4}) for each reference position is determined, and,
e) insofar as the ascertained deviations (A_{X3}, A_{X4}) themselves are different by a specified value, a signal is emitted indicating that a correction is required.

5. The method for operating a pharmacy picking device according to one of the preceding claims, wherein depending on the signal indicating that a correction is required,
a service request is electronically communicated to a service technician and/or
the pharmacy picking device (1) is switched off.

6. The method for operating a pharmacy picking device according to one of claims 1 - 5, wherein the one or more set values (X_{1S}, X_{2S}, X_{3S}, X_{4S}, X_{5S}, X_{5S}, Z_{1S}, Z_{2S}) are provided at at least one reference position, in that the one or more set values are stored in the control device (X) as default values.

7. The method for operating a pharmacy picking device according to one of claims 1 - 5, wherein the one or more set values (X_{1S}, X_{2S}, X_{3S}, X_{4S}, X_{5S}, Z_{1S}, Z_{2S}) are provided at at least one reference position, in that the one or more set values are learned after the pharmacy picking device has initially been put into operation, in that specified reference positions are approached, and when a signal characteristic of a certain reference position is detected, a value is ascertained and a set value is ascertained therefrom and this is stored in the control device (XX).

8. The method for operating a pharmacy picking device according to one of claim 1- 5, wherein the position corresponding to a set value (X_{1S}, X_{2S}, X_{3S}, X_{4S}, X_{5S,} Z_{1S}, Z_{2S}) is approached at a first speed v1, the operator unit (20) is moved further in direction of the reference position at a second speed v2, until a signal characteristic of the reference position is detected.

9. The method for operating a pharmacy picking device according to one of claim 1 - 8, **characterised in that** on the basis of the one or more deviations one or more corresponding correction factors are ascertained, which are stored in the control device, and **in that** subsequently executed control programs take the one or more correction factors into account while controlling the operator unit.

## Revendications

1. Procédé pour exploiter un dispositif de commissionnement de pharmacie avec
au moins une travée de rayonnages (10) avec respectivement une pluralité de tablettes de rayonnage (11) s'étendant dans une direction horizontale (axe X) et une pluralité de murs de rayonnage (12) s'étendant dans une direction verticale (axe Z),
au moins un appareil de manoeuvre (20) pouvant se déplacer horizontalement et verticalement devant la travée de rayonnages (10) avec un dispositif de préhension (21) pour stocker et/ou déstocker des emballages de médicaments (22) sur les tablettes de rayonnage (11) ou à partir de celles-ci, l'appareil de manoeuvre présentant au moins un capteur (23), et
une unité de commande (30) couplée avec l'appareil de manoeuvre (20),
dans lequel, pour reconnaître un écart de positionnement de l'appareil de manoeuvre en direction horizontale (axe X)
a) au moins une valeur de consigne (X_{1S}, X_{2S}, X_{3S}, X_{4S}, X_{5S}) d'au moins une position de référence est mise à disposition
b) une position correspondant à une valeur de consigne étant approchée en direction horizontale avec l'appareil de manoeuvre (20),
c) lors de la détection d'un signal caractéristique pour une position de référence, une valeur réelle (X_{1I,} X_{2I}, X_{3I}, X_{4I}, X_{5I}) de cette position de référence étant déterminée,
d) une valeur de consigne (X_{1S}, X_{2S}, X_{3S}, X_{4S}, X_{5S}) étant comparée avec une valeur réelle (X_{1I}, X_{2I}, X_{3I}, X_{4I}, X_{5I}) correspondante ou bien deux valeurs réelles étant comparées entre elles, et un écart (A_{X1}, A_{X2}, A_{X3}, A_{X4}, A_{X15}, A_{X23}) étant déterminé, et,
e) dans la mesure où un écart dépassant une valeur de seuil est déterminé, un signal indiquant la nécessité d'une correction étant émis.

2. Procédé pour exploiter un dispositif de commissionnement de pharmacie selon la revendication 1, dans lequel, pour reconnaître un écart de positionnement de l'appareil de manoeuvre en direction verticale (axe Z)
a) au moins une valeur de consigne (Z_{1S}, Z_{2S}) d'au moins une position de référence est mise à disposition
b) une position correspondant à une valeur de consigne étant approchée avec l'appareil de manoeuvre (20),
c) lors de la détection d'un signal caractéristique pour une position de référence, une valeur réelle (Z_{1I}, Z_{2I}) de cette position de référence étant déterminée,
d) une valeur de consigne (Z_{1S}, Z_{2S}) étant comparée avec une valeur réelle (Z_{1I}, Z_{2I}) correspondante ou bien deux valeurs réelles étant comparées entre elles, et un écart (A_{Z1}, A_{Z2}, A_{Z12}) étant déterminé, et,
e) dans la mesure où un écart dépassant une valeur de seuil est déterminé, un signal indiquant la nécessité d'une correction étant émis.

3. Procédé pour exploiter un dispositif de commissionnement de pharmacie selon la revendication 1 ou 2, dans lequel
le dispositif de commissionnement de pharmacie présente au moins deux travées de rayonnages (10, 10a) parallèles, entre lesquelles l'appareil de manoeuvre (20) peut se déplacer horizontalement et verticalement et le dispositif de préhension (21) de l'appareil de manoeuvre pouvant pivoter autour de l'axe vertical (axe de rotation C),
dans lequel, pour reconnaître un écart de positionnement du dispositif de préhension (21) par rapport à l'axe de rotation C après la reconnaissance d'un écart de positionnement de l'appareil de manoeuvre en direction horizontale (axe X) au niveau de l'une des travées de rayonnages (10, 10a) [étapes a)- e) de la revendication 1], on fait tourner le dispositif de préhension de l'ordre d'une valeur C prédéterminée et
a) une deuxième position correspondant à une deuxième valeur de consigne (X_{4S}) étant approchée avec l'appareil de manoeuvre (20),
b) lors de la détection d'un signal caractéristique pour la deuxième position de référence, une valeur réelle (X_{4I}) de la position de référence étant déterminée,
c) la valeur de consigne de la deuxième position de référence (X_{4S}) étant comparée avec la valeur réelle (X_{4I}) et un écart (A_{X4}) étant déterminé, et,
d) dans la mesure où les écarts déterminés (AX3, AX4) concernant le positionnement en direction X au niveau des première et deuxième travées de rayonnages (A_{X3}, A_{X4}) dépassent des valeurs de seuil où bien diffèrent de l'ordre d'une valeur prédéterminée, un signal indiquant la nécessité d'une correction étant émis.

4. Procédé pour exploiter un dispositif de commissionnement de pharmacie selon l'une des revendications susmentionnées, dans lequel le dispositif de commissionnement de pharmacie comprend deux rails de guidage (13a, 13b) horizontaux et un rail de guidage (14) vertical, le rail de guidage vertical pouvant se déplacer le long des rails de guidage horizontaux et le dispositif de préhension pouvant se déplacer le long du rail de guidage vertical (14),
dans lequel, pour reconnaître une position en biais de l'appareil de manoeuvre
a) pour le rail de guidage inférieur et supérieur (13a, 13b), au moins une valeur de consigne (X_{1S}, X_{2S}) d'au moins une position de référence est mise à disposition
b) une position correspondant aux valeurs de consigne (X_{3S}, X_{4S}) étant approchée avec l'appareil de manoeuvre (20),
c) lors de la détection d'un signal caractéristique pour une position de référence, une valeur réelle (X_{3I}, X_{4I}) de la position de référence étant déterminée,
d) les valeurs de consigne (X_{3S}, X_{4S}) étant comparées aux valeurs réelles (X_{3I}, X_{4I}), et un écart (A_{X3}, A_{X4}) par position de référence étant déterminé, et,
e) dans la mesure où les écarts (A_{X3}, A_{X4}) déterminés diffèrent eux-mêmes d'une valeur prédéterminée, un signal indiquant la nécessité d'une correction étant émis.

5. Procédé pour exploiter un dispositif de commissionnement de pharmacie selon l'une des revendications susmentionnées, dans lequel, en fonction du signal indiquant la nécessité d'une correction
une demande de service est transmise électroniquement à un collaborateur de service, et/ou
le dispositif de commissionnement de pharmacie (1) étant mis à l'arrêt.

6. Procédé pour exploiter un dispositif de commissionnement de pharmacie selon l'une des revendications 1 - 5, dans lequel la valeur de consigne ou les valeurs de consigne (X_{1S}, X_{2S}, X_{3S}, X_{4S}, X_{5S}, X_{5S}, Z_{1S}, Z_{2S}) d'au moins une position de référence est/sont mise(s) à disposition en ce que la ou les valeur(s) de consigne sont enregistrées dans le dispositif de commande (X) en tant que valeurs prescrites.

7. Procédé pour exploiter un dispositif de commissionnement de pharmacie selon l'une des revendications 1 - 5, dans lequel la valeur de consigne ou les valeurs de consigne (X_{1S}, X_{2S}, X_{3S}, X_{4S}, X_{5S,} Z_{1S}, Z_{2S}) d'au moins une position de référence est ou sont mise (s) à disposition en ce que la valeur de consigne ou les valeurs de consigne sont apprises après la première mise en service du dispositif de commissionnement de pharmacie en ce que des positions de référence prescrites sont approchées et en ce que, lors de la détection d'un signal caractéristique pour une position de référence déterminée, on détermine une valeur, une valeur de consigne étant déterminée à partir de celle-ci et étant enregistrée dans le dispositif de commande (XX).

8. Procédé pour exploiter un dispositif de commissionnement de pharmacie selon l'une des revendications 1 - 5, dans lequel la position correspondant à une valeur de consigne (X_{1S}, X_{2S}, X_{3S}, X_{4S}, X_{5S}, Z_{1S}, Z_{2S}) est approchée à une première vitesse v1, l'appareil de manoeuvre (20) étant déplacé plus loin en direction vers la position de référence à une deuxième vitesse v2 jusqu'à ce qu'un signal caractéristique pour la position de référence soit détecté.

9. Procédé pour exploiter un dispositif de commissionnement de pharmacie selon l'une des revendications 1 - 8, **caractérisé en ce que**, sur la base du ou des écart(s) déterminé(s), on détermine un ou plusieurs facteurs de correction correspondants, ceux-ci étant enregistrés dans le dispositif de commande et des programmes de commande, exécutés par la suite lors de l'excitation de l'appareil de manoeuvre, tenant compte du ou des facteurs de correction.
